# FASCICULE DE BREVET EUROPEEN

(11) **EP 2 537 553 B1**
(45) Date de publication et mention de la délivrance du brevet: **28.08.2013**
(21) Numéro de dépôt: 12171332.5
(22) Date de dépôt: 08.06.2012
(51) Int. Cl.: A61N 1/05, A61N 1/08, G01R 33/28, H03H 1/00

(54) **Sonde pour prothèse cardiaque implantable, comprenant des moyens intégrés de protection contre les effets des champs IRM**
Sonde für implantierbare Herzprothese, die integrierte Schutzmittel gegen die Effekte von Magnetresonanz-Feldern umfasst
Probe for implantable cardiac prosthesis, comprising a built-in means for protection against the effects of MRI fields

(30) Priorité: 21.06.2011 FR 1155435
(43) Date de publication de la demande: 26.12.2012
(73) Titulaire: Sorin CRM SAS, 92140 Clamart (FR)
(72) Inventeur: Makdissi, Alaa, 75011 Paris (FR)
(74) Mandataire: Dupuis-Latour, Dominique

(56) Documents cités:
- US-A1- 2008 154 348
- US-A1- 2009 149 909
- US-A1- 2009 163 980
- US-A1- 2010 076 508

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Ces dispositifs comportent un boîtier généralement désigné "générateur", raccordé électriquement et mécaniquement à une ou plusieurs "sondes" munie(s) d'électrodes destinées à venir en contact avec le myocarde sur des sites de recueil des potentiels électriques et/ou d'application des impulsions de stimulation. Ces électrodes peuvent être des électrodes endocavitaires (placées dans une cavité du myocarde en contact avec la paroi de celui-ci), épicardiques (notamment pour définir un potentiel de référence, ou pour appliquer un choc), ou encore intravasculaires (la sonde est par exemple introduite dans le sinus coronaire jusqu'à un emplacement situé face à la paroi du ventricule gauche).

L'invention concerne plus particulièrement les techniques permettant de sécuriser ces dispositifs (générateurs et leurs sondes associées) lorsque le porteur doit être soumis à un examen par imagerie par résonance magnétique (IRM ou MRI).

En effet, un examen IRM est aujourd'hui contre-indiqué aux patients porteurs d'un stimulateur ou défibrillateur cardiaque. Plusieurs types de problèmes en sont la cause :
- un échauffement à proximité des électrodes reliant le générateur au coeur du patient ;
- les forces et couples d'attraction exercés sur le dispositif plongé dans le champ magnétique statique très élevé de l'appareil IRM ;
- un comportement imprédictible du dispositif lui-même, du fait de l'exposition à ces champs magnétiques extrêmes.

La présente invention a pour objet d'apporter une solution au premier type de problème.

Le problème d'échauffement apparaît surtout au voisinage des électrodes, situées à l'extrémité distale des sondes qui sont connectées au générateur. En effet les sondes, placées dans l'imageur IRM, se comportent comme des antennes et captent le champ radiofréquence (RF) émis par l'imageur. La fréquence de ce champ RF est égale à la fréquence de Larmor des protons, soit f = 42,56 x Bo, où B₀, en Tesla, est l'induction statique caractéristique de l'imageur. Pour des inductions statiques typiques B₀ de 1,5 T ou 3 T, les fréquences RF générées corrélativement par l'imageur sont d'environ 64 MHz et 128 MHz, respectivement.

Les sondes plongées dans ce champ RF voient dès lors circuler dans leurs conducteurs des courants induits provoquant autour des électrodes en contact avec le sang un échauffement des tissus environnants. En effet, l'échauffement au niveau des électrodes étant proportionnel à la densité de courant circulant dans celles-ci, plus la surface de l'électrode est faible, plus la densité de courant est grande et donc plus l'échauffement des tissus environnants sera élevé.

En pratique, selon la configuration relative du générateur, des sondes et de l'imageur IRM, les élévations de température constatées expérimentalement atteignent typiquement 8°C (pour des électrodes en carbone) à 12°C (pour des électrodes en métal), parfois même jusqu'à 30 °C.

Or l'élévation de température ne doit pas être supérieure à ce qui est spécifié dans la norme EN 45502-1 et ses dérivés, soit moins que 2°C. En effet, à partir de 4°C survient une mort cellulaire locale qui a pour effet immédiat, entre autres, de modifier les seuils de détection et de stimulation de manière irréversible.

Il est certes possible, comme décrit notamment par les US 2003/0204217 A1 et US 2007/0255332 A1, de prévoir un mode de mise en sécurité IRM dans lequel un circuit de protection prévu au niveau du connecteur du boîtier isole les conducteurs des circuits du générateur et met tous ces conducteurs à la masse du boîtier pour empêcher la circulation de courants parasites induits.

Mais cette manière de procéder empêche le dispositif de rester fonctionnel pendant la durée de l'examen.

Or cet examen peut se prolonger plusieurs minutes, et il est pour cette raison éminemment souhaitable que le dispositif puisse continuer à assurer sans discontinuité les fonctions de détection des potentiels de dépolarisation et de stimulation éventuelle du myocarde (pendant la durée de l'examen IRM, le dispositif est toutefois basculé dans un mode particulier de fonctionnement sécurisé, désactivant notamment les circuits sensibles aux champs magnétiques élevés tels que les circuits de télémétrie RF et les alimentations à découpage).

Il n'est donc pas suffisant, en pratique, de simplement déconnecter et/ou mettre à la masse tous les conducteurs de la sonde pendant la durée de l'examen IRM.

Une autre série de techniques proposées pour réduire les courants induits par le champ IRM reposent sur la mise en série avec le(s) conducteur(s) de sonde relié(s) à l'(aux) électrode(s), sur le chemin des courants induits, d'une impédance s'opposant au passage du courant dans une situation d'examen IRM. Il peut s'agir d'un simple bobinage série (comme dans le US 7 123 013 B2), toutefois l'atténuation, si elle est sensible, n'est généralement pas suffisante. Il a été également proposé d'intercaler dans la boucle de courant un circuit résonant du type circuit bouchon accordé sur la fréquence RF générée par l'imageur, comme décrit par exemple dans les US 2009/0204171 A1 ou US 2007/0288058 A1. Mais cette solution présente l'inconvénient de nécessiter autant de filtres que de types d'imageurs différents (64 MHz, 128 MHz, ...) dans la mesure où la fréquence caractéristique n'est pas toujours la même d'un appareil à l'autre, comme on l'a expliqué plus haut.

Le EP 2 198 917 A1 (ELA Medical) propose une autre approche, consistant, dans le cas d'une sonde bipolaire, à déconnecter l'un des conducteurs et à le relier à la masse du boîtier du générateur, de manière à ce que le conducteur ainsi relié puisse faire fonction de blindage de protection de l'autre conducteur, resté fonctionnel. Cette technique est cependant réservée aux seules sondes bipolaires ; en outre, elle nécessite de modifier la conception matérielle du générateur, afin que celui-ci puisse réaliser les commutations nécessaires de mise à la masse sur détection d'un champ IRM.

D'autres techniques encore prévoient de désactiver l'électrode par un interrupteur disposé à proximité de celle-ci et commandé sur détection par le générateur d'un champ IRM détecté par un capteur incorporé dans ce dernier, comme dans le US 2010/0106227 A1. Mais la transmission de la commande depuis le générateur jusqu'à l'interrupteur situé dans la tête de sonde, à l'autre extrémité, nécessite la présence d'un conducteur supplémentaire, d'où la nécessité de concevoir une sonde spécifique et un générateur et un connecteur également spécifiques. Il n'y a donc pas de compatibilité avec les sondes ou les générateurs existants.

Le US 2008/0154348 A1 propose un circuit de protection passive situé à l'extrémité distale de la sonde et ne nécessitant pas de conducteur de liaison supplémentaire. Le principe consiste à placer une diode PIN en parallèle avec une résistance montée en série avec l'électrode. L'inconvénient majeur de ce type de protection est que, pour compenser la chute de tension dans la diode (de l'ordre de 0,6 à 0,7 V) il est nécessaire d'appliquer des impulsions de stimulation présentant une tension beaucoup plus élevée que nécessaire, et ceci de façon permanente puisque le circuit de protection, purement passif, doit rester connecté en toutes circonstances. La perte dans la diode de protection peut ainsi correspondre à plus de la moitié de l'énergie de stimulation, avec une incidence très importante sur l'autonomie de l'implant, dont la durée de vie peut être réduite de 30 à 50 %.

Le US 2009/0149909 A1 décrit un système de protection comparable, où le conducteur de la sonde est muni à son extrémité distale d'une résistance série dont les bornes sont couplées à un commutateur de type "normalement fermé", commandé par un contrôleur analysant la tension aux bornes de cette résistance.

Ce dispositif, comme le précédent, présente non seulement l'inconvénient de créer des pertes susceptibles d'avoir une incidence sur l'autonomie de l'implant, mais également d'être sensible aux signaux parasites de forte amplitude : en effet, tout signal dont l'amplitude dépasse la tension de seuil de l'interrupteur commandé entraîne une fermeture de celui-ci et donc une neutralisation de la résistance série de protection, avec un risque corrélatif d'endommagement des tissus cardiaques.

Le but de l'invention est de remédier aux différents inconvénients rencontrés avec les circuits de protection des sondes connues, en proposant une nouvelle configuration de sonde comprenant un circuit de protection intégré qui puisse assurer en toutes circonstances une "autoprotection" de la sonde à l'encontre des effets délétères des champs IRM sans impliquer le générateur , c'est-à-dire :
- qui ne nécessite pas la détection d'un champ IRM par un capteur du générateur ;
- qui ne nécessite pas de conducteur supplémentaire ;
- par voie de conséquence, qui soit utilisable avec n'importe quel générateur, sans modification matérielle des circuits de celui-ci ;
- enfin, qui ne comporte pas de composant tel qu'une diode susceptible de provoquer des pertes substantielles d'énergie lors de l'envoi des impulsions de stimulation sur les électrodes.

Plus précisément, l'invention propose une sonde du type divulgué par le US 2008/0154348 A1 précité, c'est-à-dire comprenant, côté proximal, un connecteur apte à être relié à un générateur du dispositif médical actif implantable et, côté distal, au moins une électrode de détection/stimulation reliée à un conducteur respectif s'étendant le long du corps de sonde jusqu'au connecteur, ainsi qu'un circuit de protection contre les effets délétères d'une exposition de la sonde aux champs magnétiques susceptibles d'être rencontrés lors d'un examen d'imagerie par résonance magnétique IRM. Ce circuit de protection comprend au moins un dipôle série à impédance sélectivement modifiable, interposé entre l'électrode et l'extrémité distale du conducteur associé à cette électrode, et comportant un composant résistif monté de façon permanente entre les deux bornes du dipôle, la sonde est par ailleurs essentiellement dépourvue de conducteur spécifique reliant le circuit de protection au générateur. Le dipôle comprend en outre un interrupteur actif commandé de type normalement-ouvert, apte à l'état fermé à coupler les bornes du composant résistif. Le circuit de protection comporte un étage de commande couplé au(x) conducteur(s) et comprenant des moyens aptes à détecter la tension d'une impulsion de stimulation appliquée sur le(s) conducteur(s), et à commander sélectivement par cette tension la fermeture de l'interrupteur actif pendant une durée au moins égale à la durée de détection de l'impulsion de stimulation.

L'interrupteur actif commandé est apte à l'état fermé à court-circuiter le composant résistif. De façon caractéristique de l'invention, l'étage de commande comprend un filtre discriminateur entre impulsions de stimulation et phénomènes liés à des courants induits IRM. Ce filtre discriminateur, qui est interposé entre l'interrupteur actif et les moyens d'application de la tension de l'impulsion de stimulation, est un filtre passe-bas dont la fréquence de coupure est choisie de manière à être passant dans la bande de fréquences d'une impulsion de stimulation, comprise entre 1 MHz et 10 MHz, et bloquant dans la bande de fréquences d'un signal induit par un champ magnétique IRM.

Dans le cas d'une sonde monopolaire comprenant une unique électrode de détection/stimulation reliée à un conducteur unique respectif, l'étage de command peut être un circuit couplé entre les deux bornes du composant résistif du dipôle série à impédance sélectivement modifiable.

Dans le cas d'une sonde bipolaire comprenant deux électrodes distinctes de détection/stimulation reliées à deux conducteurs respectifs, l'étage de commande peut être un circuit couplé entre ces deux conducteurs.

Dans un mode de réalisation particulier adapté à un fonctionnement avec une impulsion de stimulation monophasique, l'étage de commande comprend un condensateur apte à être chargé par la tension de l'impulsion de stimulation, et une résistance de décharge de ce condensateur. Cet ensemble condensateur-résistance présente une constante de temps propre à commander sélectivement la fermeture de l'interrupteur actif pendant un temps au moins égal à la durée nominale de décharge du condensateur de l'étage de sortie du générateur auquel la sonde est destinée à être reliée.

Dans ce cas, la sonde comprend des moyens de charge du condensateur par la tension de l'impulsion de stimulation, avec un dipôle redresseur comprenant avantageusement un transistor dont la conduction du canal est commandée par la sortie d'un comparateur dont les bornes d'entrée sont couplées aux deux bornes de ce dipôle redresseur.

Dans un mode de réalisation avec sonde bipolaire adapté à un fonctionnement avec une impulsion de stimulation monophasique, l'étage de commande comprend un circuit de type minimum-maximum ou valeur-absolue couplé entre les deux conducteurs, apte à délivrer une tension de signe constant pendant la durée de l'impulsion de stimulation, et un condensateur apte à être chargé par la tension délivrée par le circuit de type minimum-maximum ou valeur-absolue.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une vue schématique d'un générateur et d'une sonde munie du circuit de protection selon l'invention.
La Figure 2 est une représentation schématique fonctionnelle du circuit de protection selon l'invention.
La Figure 3 illustre un premier mode de réalisation du circuit de protection, adapté à une sonde bipolaire.
La Figure 4 est une série de chronogrammes montrant les variations de différents paramètres du circuit de la Figure 3 lors de l'application d'une impulsion de stimulation.
La Figure 5 illustre une forme de réalisation avantageuse de l'étage redresseur du circuit de commande des interrupteurs, permettant de minimiser la chute de tension, et donc les pertes électriques, à ce niveau.
La Figure 6 illustre un mode de réalisation simplifié de l'invention, adapté à une sonde monopolaire et une stimulation par impulsion monophasique.

On va maintenant décrire un exemple de réalisation du dispositif de l'invention.

De façon générale, on notera que la configuration sécurisée selon l'invention peut être utilisée non seulement pour les examens IRM, mais également comme protection dans divers autres environnements électromagnétiques créés par des appareils médicaux tels que bistouris électriques, dispositifs de stimulation électrique transcutanée des nerfs (TENS), etc. ou encore des équipements de la vie courante tels que portiques antivol, dispositifs de surveillance électrique des articles (EAS), etc.

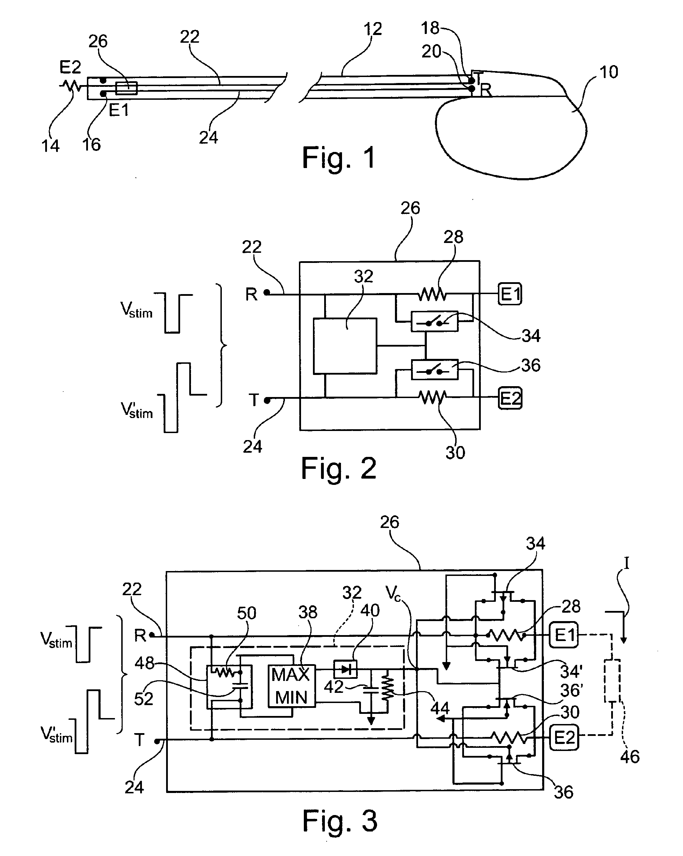

Sur la Figure 1, on a illustré de façon très schématique un dispositif médical implantable, comprenant un générateur 10 muni de sa sonde 12.

Dans l'exemple illustré il s'agit d'une sonde bipolaire comprenant à son extrémité distale une première électrode E2, qui est une électrode distale d'extrémité ou électrode *tip* (T) de faible surface, par exemple une vis d'ancrage 14 dans la paroi cardiaque. L'autre électrode E1 est par exemple une électrode annulaire 16 ou électrode *ring* (R).

Les deux électrodes E1 et E2, situées à l'extrémité distale de la sonde 12, sont reliées par des conducteurs respectifs 22, 24, s'étendant sur toute la longueur du corps de sonde, à des pôles 18, 20 d'un connecteur situé à l'extrémité proximale et relié à deux bornes respectives T et R du générateur 10. Le connecteur (non représenté) permet le couplage mécanique et électrique de la sonde au générateur implanté 12.

La sonde est pourvue d'un circuit de protection 26 disposé à son extrémité distale, à proximité des deux électrodes E1 et E2.

La Figure 2 illustre de façon schématique les éléments de ce circuit de protection 26.

Ce circuit 26 est intercalé entre, d'une part, les électrodes E1 et E2 et, d'autre part, les conducteurs 22 et 24.

Le circuit 26 comporte une résistance 28 intercalée en permanence entre l'électrode E1 et le conducteur 22, et de même une résistance 30 intercalée en permanence entre l'électrode E2 et le conducteur correspondant 24. Ces résistances 28, 30 présentent par exemple une valeur de l'ordre de 20 kΩ, suffisante pour assurer une protection permanente à l'encontre des courants excessifs qui pourraient être induits dans les conducteurs par un champ IRM. Cette valeur rend d'autre part possible la détection en toutes circonstances des potentiels cardiaques par les électrodes E1 ou E2, en présence ou en l'absence de champ IRM du fait de la haute impédance, de l'ordre de quelques mégohms, de l'étage de mesure des potentiels cardiaques.

Pour la stimulation, il est toutefois nécessaire de désactiver ces résistances 28, 30. Cette fonction est réalisée par un étage de commande 32 dont le rôle est de recueillir et conserver la tension de stimulation apparaissant entre les conducteurs 22 et 24 et d'utiliser cette tension pour commander des interrupteurs actifs 34, 36 permettant de court-circuiter les résistances 28, 30 respectives, permettant ainsi le passage du courant de stimulation sans pertes dans le circuit de protection 26.

Les interrupteurs actifs commandés 34, 36 sont des interrupteurs à l'état solide, avantageusement réalisés en technologie MOS, notamment CMOS, et leur état est un état "normalement ouvert" c'est-à-dire que, du point de vue de la circulation du courant, la résistance 28, 30 est toujours en série dans la boucle de courant de l'électrode, sauf dans les périodes où les interrupteurs 34 et 36 sont commandés par l'étage de commande 32.

Dans le cas d'une impulsion de stimulation monophasique telle que l'impulsion Vₛₜᵢₘ illustrée, il est nécessaire de désactiver les résistances 28, 30 non seulement pendant la durée d'application de l'impulsion Vₛₜᵢₘ aux tissus cardiaques, mais également pendant une durée suffisante pour permettre la décharge du condensateur de sortie de l'étage de stimulation du générateur (fonction dite OCD, *Output Capacitor Discharge*). La largeur de l'impulsion Vₛₜᵢₘ étant de l'ordre de 100 µs à 1 ms, et la durée de la phase OCD étant de l'ordre de 20 ms, l'étage de commande 32 doit mémoriser la tension de l'impulsion de stimulation de manière à activer et maintenir fermés les interrupteurs 34, 36 pendant toute la durée de cette phase OCD.

En revanche, dans le cas d'une impulsion de stimulation biphasique telles que l'impulsion Vₛₜᵢₘ illustrée, l'inversion de polarité rend inutile la mise en oeuvre d'une fonction OCD, la décharge du condensateur de sortie intervenant d'elle-même du fait de l'inversion de polarité.

L'impédance globale de la sonde, vue depuis le connecteur 18, 20 du générateur, peut ainsi prendre deux valeurs différentes :
- une valeur faible, de l'ordre de 20 Ω, pendant la durée d'une impulsion de stimulation et la durée de la phase OCD immédiatement consécutive, et
- une valeur élevée, typiquement 20 kΩ, le reste du temps.

La Figure 3 donne un exemple de réalisation d'un tel circuit de protection 26, adapté à une sonde bipolaire et une stimulation par impulsion monophasique.

Dans cette réalisation, les résistances 28, 30 sont court-circuitées chacune par une paire de transistors complémentaires CMOS, respectivement 34, 34' et 36, 36'. Les grilles de ces transistors sont commandées par une tension de commande V_{c} délivrée par l'étage de commande 32. L'étage de commande 32 comprend tout d'abord un filtre passe-bas 48, par exemple un filtre du premier ordre comprenant une résistance 50 et un condensateur 52. La fréquence de coupure de ce filtre est calculée de manière qu'il soit passant dans la bande de fréquence d'une impulsion de stimulation (typiquement comprise entre 1 MHz et 10 MHz) et en revanche bloquant dans la bande de fréquence correspondant à un signal induit par un champ magnétique IRM. Ce filtre permet donc de bloquer les signaux parasites IRM et de laisser passer les impulsions de stimulation.

En aval de ce filtre passe-bas 48 est disposé un circuit 38 de type "minimum-maximum" ou "valeur absolue", qui permet de délivrer en sortie une tension toujours positive, quelle que soit la polarité de l'impulsion de stimulation appliquée en entrée du circuit 26 et qui aura traversé le filtre passe-bas 48. Cette tension de sortie est utilisée pour charger, via un étage redresseur 40, un condensateur 42 aux bornes duquel est définie la tension de commande V_{c} servant à piloter les interrupteurs commandés 34, 34' et 36, 36'.

Une résistance de décharge 44 est montée aux bornes du condensateur 42, et sa valeur est calculée de manière que la constante de temps RC, qui détermine le temps de décharge du condensateur 42 après la disparition de l'impulsion de stimulation Vₛₜᵢₘ, reste au-dessus du seuil de commande des interrupteurs MOS 34, 34', 36, 36' pendant une durée suffisante pour permettre la décharge OCD de l'étage de sortie du générateur. En d'autres termes, cette constante de temps RC permet de fixer, par une solution matérielle simple, le temps de maintien de la commande de fermeture des interrupteurs actifs de court-circuit des résistances 28, 30. L'étage de commande 32 permet ainsi de discriminer (via le filtre 48) entre les signaux IRM indésirables et les signaux correspondant à une impulsion de stimulation cardiaque. Dans ce dernier cas, cet étage stocke dans le condensateur 42, via le circuit minimum-maximum 38 et le redresseur 40, une fraction extrêmement réduite de l'énergie de stimulation. La tension V_{c} aux bornes de ce condensateur 42 permet de commander les interrupteurs qui court-circuitent les résistances 28 et 30, et ceci pendant un temps limité, défini par la résistance 44 (constante de temps de décharge du condensateur 42).

Le filtre discriminateur 48 permet d'éviter de déclencher la fermeture de ces interrupteurs dans le cas d'un signal IRM parasite dont l'amplification dépasserait la tension de seuil des interrupteurs 34, 34', 36, 36', de l'ordre de 1 V environ.

D'autre part, la résistance 44 de décharge du condensateur 42 évite de maintenir les interrupteurs fermés pendant une longue période après une stimulation, ce qui risquerait, pendant cette période, d'ouvrir le passage à un quelconque signal parasite vers le coeur.

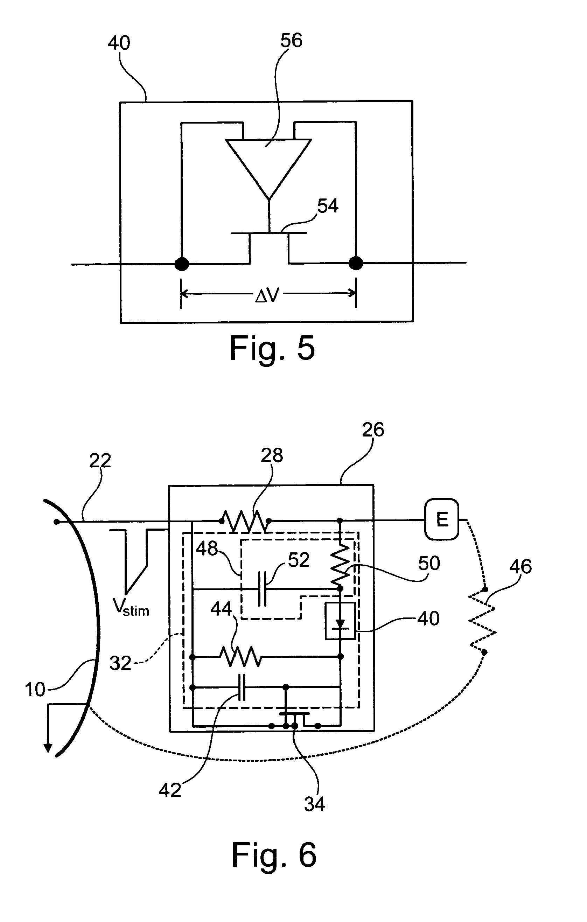

L'étage redresseur 40 peut être réalisé simplement sous la forme d'une diode. Il peut être également, de façon plus avantageuse, réalisé de la manière illustrée Figure 5, avec un transistor 54 de type MOS dont la conduction du canal est commandée par la sortie d'un comparateur 56 dont les bornes d'entrée sont couplées aux deux bornes de ce transistor MOS 54. Selon la polarité des potentiels aux bornes du transistor 54, le comparateur délivre ou ne délivre pas en sortie un signal de déblocage de ce transistor, ce qui permet de le rendre passant dans un sens et bloquant dans l'autre. Par rapport à une simple diode, cette configuration permet de n'avoir aux bornes dudit pôle redresseur 40 qu'une chute de tension ΔV très faible, de l'ordre de 0,1 V, bien moindre que la chute de tension directe d'une simple diode, de l'ordre de 0,7 V. Les pertes électriques en seront réduites d'autant (on se souviendra que ce circuit est alimenté par la seule énergie des impulsions de stimulation, énergie qu'il convient de ménager pour ne pas obérer la durée de vie de l'implant).

On notera que, de façon générale, le circuit que l'on a illustré sur cette Figure 3 peut fonctionner indifféremment avec des impulsions monophasiques Vₛₜᵢₘ (et ceci quelle que soit la polarité de l'impulsion) ou bien biphasiques Vₛₜᵢₘ.

Dans le cas d'une stimulation exclusivement par impulsions monophasiques, la polarité de celles-ci est connue, car la stimulation est toujours opérée en polarité négative. Il n'est donc pas nécessaire de prévoir dans ce cas un circuit 38 de type maximum-minimum ou valeur absolue, et le condensateur 42 peut être chargé directement par la tension présente en sortie du filtre passe-bas 48.

Dans le cas d'une stimulation exclusivement par impulsions biphasiques, le circuit 38 est en revanche indispensable pour neutraliser l'effet de l'inversion de polarité, mais inversement la fonction de décharge du condensateur 42 (fonction OCD permise par la résistance 44) n'est plus nécessaire car les charges accumulées à l'interface coeur/électrode seront annulées du fait de l'inversion de polarité de l'impulsion de stimulation appliquée à cet endroit.

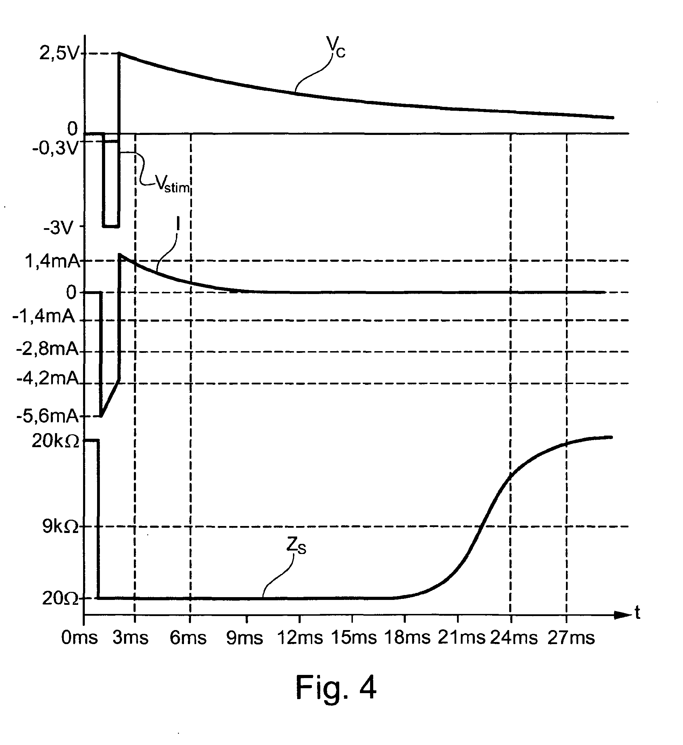

La Figure 4 est une série de chronogrammes montrant les variations de différents paramètres du circuit de la Figure 3 lors de l'application d'une impulsion de stimulation.

Sur le chronogramme du haut, on a représenté l'impulsion de stimulation Vₛₜᵢₘ et la tension de commande V_{c} délivrée par l'étage de commande 32, qui décroît progressivement du fait de la constante de temps RC, de l'ordre de 15 ms, de l'ensemble condensateur 42-résistance 44.

Le chronogramme du milieu illustre le courant I passant dans les tissus cardiaques, modélisés par l'impédance 46 sur la Figure 3 (impédance corporelle), de l'ordre de 500 Ω.

Le chronogramme du bas indique la variation de l'impédance globale Zₛ de la sonde, vue depuis le générateur :
- en temps normal, c'est-à-dire juste avant l'application de l'impulsion de stimulation, cette impédance est élevée (20 kΩ) assurant une protection permanente à l'encontre d'éventuels courants induits par un examen IRM, notamment ;
- à l'instant d'application de l'impulsion de stimulation Vₛₜᵢₘ, cette impédance chute quasi-instantanément à une faible valeur, de l'ordre de 20 Ω, du fait de la fermeture des interrupteurs commandés actifs 34, 34', 36 et 36'. Cette faible impédance est maintenue pendant une durée de l'ordre de 20 ms au moins, nécessaire à la décharge OCD suivant l'application de l'impulsion de stimulation.

Du point de vue de la réalisation matérielle, on notera que les circuits actifs peuvent être réalisés en technologie CMOS standard, permettant d'intégrer dans un même circuit spécifique de type ASIC tous les composants du circuit de protection 26 (y compris le condensateur 42). Ce circuit peut présenter de très faibles dimensions, typiquement inférieures à 0,4 x 0,4 mm, ce qui permet de le loger dans la tête de sonde, à proximité des électrodes.

La Figure 6 illustre un mode de réalisation simplifié de l'invention, adapté au cas d'une sonde monopolaire avec stimulation par impulsion monophasique.

La sonde monopolaire ne comporte (fonctionnellement tout au moins) qu'une seule électrode E, reliée par un conducteur 22 à l'une des bornes du générateur. La fermeture de la boucle de courant s'effectue par la masse du boîtier du générateur 10, comme illustré en tiretés sur la Figure 6.

Le circuit de protection 26 comprend toujours la résistance 28 intercalée en série entre le conducteur 22 et l'électrode E, et l'interrupteur actif normalement ouvert 34 permettant de court-circuiter sélectivement cette résistance 28.

Il n'est plus possible dans ce cas de surveiller les variations de tension entre les deux bornes R et T comme dans le mode de réalisation des Figures 2 et 3, puisque le potentiel du boîtier 10 n'est pas accessible directement par le circuit 26, qui n'y est pas relié.

La surveillance des variations de tension se fait alors aux bornes de la résistance série 28 du circuit de protection : si la chute de tension aux bornes de cette résistance est supérieure à la tension de seuil d'une diode 52, on dispose alors d'une tension de commande V_{c} qui pourra être utilisée, comme précédemment, pour charger un condensateur 42 (associé à une résistance de décharge 44). La tension V_{c} aux bornes du condensateur 42 n'est pas appliquée directement à la grille de l'interrupteur 34, mais via le filtre passe-bas 48 constitué, comme dans le mode de réalisation de la figure 3, d'une résistance 52 et d'un condensateur 54. La constante de temps RC de ce filtre passe-bas 52, 54 est, ici encore, choisie de l'ordre de 10 µs, correspondant à une fréquence de coupure de 20 kHz permettant d'assurer de façon sûre une discrimination entre une impulsion de stimulation (qui ne contient essentiellement que des composantes basse fréquence) et un phénomène lié à un courant induit IRM (avec des composantes essentiellement à haute fréquence).

Dans le premier cas (impulsion de stimulation), et seulement dans ce cas, la tension de l'énergie de stimulation peut être transmise au condensateur 42 via l'étage redresseur 40, pour charger ce condensateur par une faible fraction de l'impulsion de stimulation. La tension de commande V_{c} aux bornes de ce condensateur est transmise à l'interrupteur 34, qui court-circuite la résistance 28 et abaisse ainsi l'impédance globale de la sonde, de 20 kΩ environ à 20 Ω environ, comme dans les modes de réalisation précédents. Cet abaissement d'impédance est opéré pendant une durée suffisante pour permettre la décharge OCD de l'étage de sortie du générateur 10, durée déterminée par la constante de temps RC du condensateur 42 et de sa résistance de décharge 44.

Ici encore, pour éviter les pertes au sein du circuit de protection, l'étage redresseur 40 peut être réalisé sous la forme illustrée Figure 5, sous forme d'un transistor contrôlé par un comparateur, en lieu et place d'une simple diode.

## Revendications

1. Une sonde de détection/stimulation pour un dispositif médical actif implantable du type prothèse cardiaque de stimulation, resynchronisation et/ou défibrillation, comprenant :
- côté proximal, un connecteur apte à être relié à un générateur (10) du dispositif médical actif implantable ;
- côté distal :
· au moins une électrode (E1, E2 ; E) de détection/stimulation, reliée à un conducteur respectif (22, 24 ; 22) s'étendant le long du corps de sonde (12) jusqu'au connecteur, et
· un circuit (26) de protection contre les effets délétères d'une exposition de la sonde aux champs magnétiques susceptibles d'être rencontrés lors d'un examen d'imagerie par résonance magnétique IRM, ce circuit de protection comprenant au moins un dipôle série à impédance sélectivement modifiable, interposé entre l'électrode (E1, E2 ; E) et l'extrémité distale du conducteur (22, 24 ; 22) associé à cette électrode, et comportant un composant résistif (28) monté de façon permanente entre les deux bornes du dipôle,
cette sonde étant essentiellement dépourvue de conducteur spécifique reliant le circuit de protection au générateur,
sonde dans laquelle :
- le dipôle comprend en outre un interrupteur actif commandé (34, 36 ; 34) de type normalement-ouvert, apte à l'état fermé à coupler les bornes du composant résistif, et
- le circuit de protection comporte un étage de commande (32) couplé au(x) conducteur(s) et comprenant des moyens (38-44 ; 48, 50 ; 42-54) aptes à détecter la tension d'une impulsion de stimulation appliquée sur le(s) conducteur(s), et à commander sélectivement par cette tension la fermeture de l'interrupteur actif pendant une durée au moins égale à la durée de détection de l'impulsion de stimulation,
- l'interrupteur actif commandé (34, 36 ; 34) est apte à l'état fermé à court-circuiter le composant résistif,
sonde **caractérisée en ce que**:
- ledit étage de commande (32) comprend un filtre discriminateur (48) entre impulsions de stimulation et phénomènes liés à des courants induits IRM,
ce filtre discriminateur (48) étant interposé entre l'interrupteur actif (34) et les moyens d'application de la tension de l'impulsion de stimulation, et
ce filtre discriminateur (48) étant un filtre passe-bas (50, 52) dont la fréquence de coupure est choisie de manière à être passant dans la bande de fréquences d'une impulsion de stimulation, comprise entre 1 MHz et 10 MHz, et bloquant dans la bande de fréquences d'un signal induit par un champ magnétique IRM.

2. La sonde de la revendication 1, dans laquelle :
- la sonde est une sonde monopolaire comprenant une unique électrode (E) de détection/stimulation reliée à un conducteur unique (22) respectif, et
- l'étage de commande (32) est un circuit couplé entre les deux bornes dudit composant résistif (28) du dipôle série à impédance sélectivement modifiable.

3. La sonde de la revendication 1, dans laquelle :
- la sonde est une sonde bipolaire comprenant deux électrodes distinctes (E1, E2) de détection/stimulation reliées à deux conducteurs (22, 24) respectifs, et
- l'étage de commande (32) est un circuit couplé entre lesdits deux conducteurs (22, 24).

4. La sonde de la revendication 1, adaptée à un fonctionnement avec une impulsion de stimulation monophasique, dans laquelle l'étage de commande (32) comprend :
- un condensateur (42) apte à être chargé par la tension de l'impulsion de stimulation, et
- une résistance (44) de décharge de ce condensateur,
cet ensemble condensateur-résistance présentant une constante de temps propre à commander sélectivement la fermeture de l'interrupteur actif pendant un temps au moins égal à la durée nominale de décharge du condensateur de l'étage de sortie du générateur auquel la sonde est destinée à être reliée.

5. La sonde de la revendication 4, comprenant des moyens de charge dudit condensateur (42) par la tension de l'impulsion de stimulation, ces moyens de charge comprenant un dipôle redresseur (40) avec un transistor (54) dont la conduction du canal est commandée par la sortie d'un comparateur (26) dont les bornes d'entrée sont couplées aux deux bornes de ce dipôle redresseur.

6. La sonde de la revendication 1, adaptée à un fonctionnement avec une impulsion de stimulation biphasique, dans laquelle l'étage de commande (32) comprend :
- un circuit (38) de type minimum-maximum ou valeur-absolue, couplé entre les deux conducteurs (22, 24), apte à délivrer une tension (Vc) de signe constant pendant la durée de l'impulsion de stimulation, et
- un condensateur (42) apte à être chargé par la tension délivrée par le circuit de type minimum-maximum ou valeur-absolue.

## Patentansprüche

1. Eine Sonde zum Erfassen/Stimulieren für eine aktive medizinische implantierbare Vorrichtung von der Art einer Herzprothese zur Stimulation, Resynchronisation und/oder Defibrillation, aufweisend:
- proximal angeordnet, einen Stecker, der geeignet ist, um mit einem Generator (10) der aktiven medizinischen implantierbaren Vorrichtung verbunden zu sein;
- distal angeordnet:
o zumindest eine Elektrode (E1, E2; E) zum Erfassen/Stimulieren, die mit einem jeweiligen Leiter (22, 24; 22) verbunden ist, der sich entlang des Körpers der Sonde (12) bis zum Stecker erstreckt, und
o einen Schutzschaltkreis (26) gegen die schädlichen Effekte einer Explosion der Sonde auf die magnetischen Felder, die möglicherweise während einer Bildgebungsuntersuchung durch magnetische Resonanz (IRM) angetroffen werden können, wobei dieser Schutzschaltkreis zumindest einen wahlweise modifizierbaren Serienimpendanzdipol aufweist, der eingesetzt ist zwischen der Elektrode (E1, E2; E) und dem distalen Ende des Leiters (22, 24; 22), der dieser Elektrode zugeordnet ist, und eine Widerstandskomponente (28) aufweist, die auf permanente Weise zwischen die zwei Anschlüsse des Dipols montiert ist,
diese Sonde ist im Wesentlichen frei von einem spezifischen Leiter, der den Schutzschaltkreis mit dem Generator verbindet,
wobei:
- der Dipol unter anderem einen aktiven gesteuerten Schalter (34, 36; 34) der normalerweise offenen Art aufweist, der geeignet ist, um im geschlossenen Zustand die Anschlüsse der Widerstandskomponente zu koppeln, und
- der Schutzschaltkreis eine Steuerungsstufe (32) aufweist, die mit dem oder den Leitern gekoppelt ist und Mittel (38 bis 44; 48, 50; 42 bis 54) aufweist, die geeignet sind, um die Spannung eines Stimulationsimpulses zu erfassen, welcher auf den oder die Leiter aufgebracht wurde, und um wahlweise durch diese Spannung das Schließen des aktiven Schalters während einer Zeitdauer zu steuern, die zumindest gleich der Zeitdauer der Erfassung des Stimulationsimpulses ist,
- der aktive gesteuerte Schalter (34, 36; 34) im geschlossenen Zustand geeignet ist, um die Widerstandskomponente kurzzuschließen,
wobei die Sonde **dadurch gekennzeichnet ist, dass**
- die Steuerstufe (32) einen Diskriminatorfilter (48) zwischen Stimulationsimpulsen und Phänomenen umfasst, die sich auf induzierte IRM-Ströme beziehen,
dieser Diskriminatorfilter (48) ist zwischen dem aktiven Schalter (34) und den Aufbringungsmitteln der Spannung des Stimulationsimpulses eingesetzt, und
dieser Diskriminatorfilter (48) ist ein Tiefpassfilter (50, 52), dessen Grenzfrequenz so ausgewählt ist, dass er in dem Frequenzband eines Stimulationsimpulses durchlässig ist, zwischen 1 MHz und 10 MHz liegend, und in dem Frequenzband eines durch ein IRM magnetisches Feld induzierten Signals blockiert.

2. Sonde nach Anspruch 1, in der:
- die Sonde eine monopolare Sonde ist, die eine einzige Elektrode (E) zum Erfassen/Stimulieren aufweist, die mit einem entsprechenden einzigen Leiter (22) verbunden ist, und
- die Steuerungsstufe (32) ein Schaltkreis ist, der zwischen den zwei Anschlüssen der Widerstandskomponente (28) des wahlweise modifizierbaren Serienimpedanzdipols gekoppelt ist.

3. Sonde nach Anspruch 1, in der:
- die Sonde eine bipolare Sonde ist, die zwei beabstandete Elektroden (E1, E2) zum Erfassen/Stimulieren aufweist, die mit zwei entsprechenden Leitern (22, 24) verbunden sind, und
- die Steuerungsstufe (32) ein Schaltkreis ist, der zwischen die zwei Leiter (22, 24) gekoppelt ist.

4. Sonde nach Anspruch 1, die für einen Betrieb mit einem Einzelphasenstimulationsimpuls geeignet ist, in der die Steuerungsstufe (32) aufweist:
- einen Kondensator (42), der geeignet ist, durch die Spannung des Stimulationsimpulses geladen zu werden, und
- einen Widerstand (44) zum Entladen dieses Kondensators,
wobei diese Kondensator-Widerstandsgesamtheit eine eigene Zeitkonstante zum wahlweisen Steuern des Schließens des aktiven Schalters während einer Zeit zumindest gleich der nominalen Dauer des Entladens des Kondensators der Ausgangsstufe des Generators darstellt, mit dem die Sonde verbunden werden soll.

5. Sonde nach Anspruch 4, die Mittel zum Laden des Kondensators (42) durch die Spannung des Stimulationsimpulses aufweist, wobei diese Lademittel einen Gleichrichterdipol (40) mit einem Transistor (54) aufweist, dessen Kanalleitung durch den Ausgang eines Komparators (26) geregelt wird, dessen Eingangsanschlüsse mit den zwei Anschlüssen dieses Gleichrichterdipols gekoppelt sind.

6. Sonde nach Anspruch 1, die angepasst ist für einen Betrieb mit einem zweiphasigen Stimulationsimpuls, in der die Steuerstufe (32) aufweist:
- einen Schaltkreis (38) der Minimum-Maximum oder Absolutwertart, der zwischen die zwei Leiter (22, 24) gekoppelt ist, und geeignet, um eine Spannung (Vc) konstanten Vorzeichens während der Dauer des Stimulationsimpulses bereitzustellen, und
- einen Kondensator (42), der geeignet ist, um geladen zu werden durch die Spannung, die durch den Schaltkreis der Minimum-Maximum oder Absolutwertart bereitgestellt ist.

## Claims

1. Detection/stimulation lead for an implantable active medical device of the stimulation, resynchronization and/or defibrillation cardiac prosthesis type, comprising:
- at the proximal end, a connector that can be connected to a generator (10) of the implantable active medical device;
- at the distal end:
• at least one detection/stimulation electrode (E1, E2; E), connected to a respective conductor (22, 24; 22) extending along the lead body (12) to the connector, and
• a protection circuit (26) for protection against the damaging effects of exposure of the lead to the magnetic fields likely to be encountered during a magnetic resonance imaging MRI examination, this protection circuit comprising at least one series dipole with selectively modifiable impedance, interposed between the electrode (E1, E2; E) and the distal end of the conductor (22, 24; 22) associated with this electrode, and comprising a resistive component (28) mounted permanently between the two terminals of the dipole,
this lead essentially having no specific conductor connecting the protection circuit to the generator, wherein:
- the dipole also comprises a controlled active switch (34, 36; 34) of normally-open type, suited in its closed state to couple the terminals of the resistive component, and
- the protection circuit comprises a control stage (32) coupled to the conductor(s) and comprising means (38-44; 48, 50; 42-54) which can detect the voltage of a stimulation pulse applied to the conductor(s), and selectively control, by this voltage, the closing of the active switch for a duration at least equal to the duration of detection of the stimulation pulse,
- the controlled active switch (34, 36; 34) is suited in its closed state to short-circuit the resistive component,
the lead being **characterized in that**:
- said control stage (32) comprises a discriminating filter (48) discriminating between stimulation pulses and phenomena linked to MRI-induced currents,
this discriminating filter (48) being interposed between the active switch (34) and the means for applying the stimulation pulse voltage, and
this discriminating filter (48) being a low-pass filter (50, 52) with a cut-off frequency which is chosen to be in the passing state in the frequency band of a stimulation pulse, between 1 MHz and 10 MHz, and in the blocking state in the frequency band of a signal induced by an MRI magnetic field.

2. Lead according to Claim 1, in which:
- the lead is a monopolar lead comprising a single detection/stimulation electrode (E) connected to a respective single conductor (22), and
- the control stage (32) is a circuit coupled between the two terminals of said resistive component (28) of the series dipole with selectively modifiable impedance.

3. Lead according to Claim 1, in which:
- the lead is a bipolar lead comprising two distinct detection/stimulation electrodes (E1, E2) connected to two respective conductors (22, 24), and
- the control stage (32) is a circuit coupled between said two conductors (22, 24).

4. Lead according to Claim 1, suitable for operation with a single-phase stimulation pulse, in which the control stage (32) comprises:
- a capacitor (42) which can be charged by the stimulation pulse voltage, and
- a discharge resistor (44) for this capacitor, this capacitor-resistor assembly exhibiting a time constant suitable for selectively controlling the closing of the active switch for a time at least equal to the nominal discharge time of the capacitor of the output stage of the generator to which the lead is intended to be linked.

5. Lead according to Claim 4, comprising means for charging said capacitor (42) by the stimulation pulse voltage, said charging means comprising a rectifying dipole (40) with a transistor (54) in which the conduction of the channel is controlled by the output of a comparator (26) whose input terminals are coupled to the two terminals of said rectifying dipole.

6. Lead according to Claim 1, suitable for operation with a biphasic stimulation pulse, in which the control stage (32) comprises:
- a circuit (38) of minimum-maximum or absolute value type, coupled between the two conductors (22, 24), suitable for delivering a voltage (Vc) of constant sign for the duration of the stimulation pulse, and
- a capacitor (42) suitable for charging by the voltage delivered by said circuit of minimum-maximum or absolute value type.
